Europäisches Patentamt
European Patent Office
Office européen des brevets

(19) (11) Veröffentlichungsnummer: **0 371 925 A2**

(12) **EUROPÄISCHE PATENTANMELDUNG**

(21) Anmeldenummer: **89810892.3**

(22) Anmeldetag: **22.11.89**

(51) Int. Cl.[5]: **C07D 417/04, C07D 413/04, A01N 43/82**

Claim for the following Contracting State: ES.

Die Bezeichnung der Erfindung wurde geändert (Richtlinien für die Prüfung im EPA, A-III, 7.3).

(30) Priorität: **01.12.88 CH 4458/88**

(43) Veröffentlichungstag der Anmeldung: **06.06.90 Patentblatt 90/23**

(84) Benannte Vertragsstaaten: **AT BE CH DE ES FR GB GR IT LI LU NL**

(71) Anmelder: **CIBA-GEIGY AG**
**Klybeckstrasse 141**
**CH-4002 Basel(CH)**

(72) Erfinder: **Eckhardt, Wolfgang, Dr.**
**Breslauerstrasse 14**
**D-7850 Lörrach(DE)**
Erfinder: **Beriger, Ernst, Dr.**
**Grabenmattweg 29**
**CH-4123 Allschwil(CH)**
Erfinder: **Zondler, Helmut, Dr.**
**Oberwilerstrasse 49**
**CH-4103 Bottmingen(CH)**

(54) **2-Mercapto-5-pyrimidyl-1,3,4-oxadiazol-Derivate mit nematizider und fungizider Wirkung.**

(57) Es werden 2-Mercapto-5-pyrimidyl-1,3,4-oxadiazole und 2-Mercapto-5-pyrimidyl-1,3,4-thiadiazole der Formel I

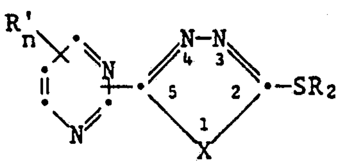

(I)

in welcher
X Sauerstoff oder Schwefel,
$R'$ Halogen, $CF_3$, $C_1$-$C_3$-Alkyl, $C_1$-$C_3$-Alkoxy, $C_1$-$C_3$-Alkylthio, Cyclopropyl und n eine Zahl von 0 bis 3,
$R_2$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, bedeuten sowie Verfahren zur Herstellung der Verbindungen der Formel I beschrieben.

Die Verbindungen der Formel I besitzen nematizide und fungizide Eigenschaften. Es werden nematizide und fungizide Mittel, die als Wirkstoff mindestens einen Wirkstoff der Formel I enthalten, ferner Verfahren zur Verwendung der Wirkstoffe und der Mittel bei der Bekämpfung von Nematoden oder Pilzen beschrieben.

EP 0 371 925 A2

## Nematizide und fungizide Mittel

Die vorliegende Erfindung betrifft neue substituierte 2-Mercapto-5-pyrimidyl-1,3,4-oxa- und -thiadiazole, deren Herstellung sowie nematizide und fungizide Mittel, die als Wirkstoff mindestens eine dieser Verbindungen enthalten. Die Erfindung betrifft ferner neue Zwischenprodukte des Verfahrens zur Herstellung der Wirkstoffe, die Verwendung von 2-Mercapto-5-pyrimidyl-1,3,4-oxa- und -thiadiazolen und Mittel zur Bekämpfung von Nematoden, insbesondere von pflanzenschädigenden Nematoden, sowie Pilzen, insbesondere von pflanzenschädigenden bodenbürtigen Pilzen.

Die erfindungsgemässen 2-Mercapto-5-pyrimidyl-1,3,4-oxadiazole und 2-Mercapto-5-pyrimidyl-1,3,4-thiadiazole entsprechen der allgemeinen Formel I

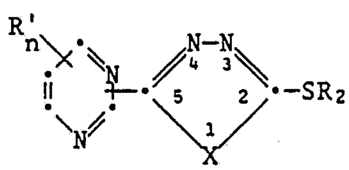

(I)

in welcher

X Sauerstoff oder Schwefel

R′ Halogen, $CF_3$, $C_1$-$C_3$-Alkyl, $C_1$-$C_3$-Alkoxy, $C_1$-$C_3$-Alkylthio, Cyclopropyl und n eine Zahl von 0 bis 3,

$R_2$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, bedeuten

sowie Salze dieser Verbindungen.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die normalen und isomeren Propyl-, Butyl- und Pentylgruppen. Halogensubstituiertes Alkyl steht für einen einfach bis perhalogenierten Alkylrest, wie z.B. $CHCl_2$, $CH_2F$, $CCl_3$, $CH_2Cl$; $CHFCH_3$, $CH_2CH_2Br$, $CF_2CF_3$, $C_2Cl_5$, $CH_2Br$, $CHBrCl$ usw., vorzugsweise für $CHF_2$. Alkenyl bedeutet z.B. Propenyl-(1), Allyl, Butenyl-(1), Butenyl-(2) oder Butenyl-(3) sowie Ketten mit mehreren Doppelbindungen. Halogensubstituiertes Alkenyl steht beispielsweise für 2-Brom-2-propen-1-yl oder vorzugsweise für 3,4,4-Trifluor-3-buten-1-yl, Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Fluor, Chlor oder Brom.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Diese bekannten Verbindungen haben bisher als Nematizide die in der Praxis an sie gestellten Ansprüche nicht in vollem Umfang befriedigen können. Ferner sind Oxadiazol-Derivate mit fungizider Wirksamkeit in der DE-OS 2 361 613 beschrieben. Es werden darin jedoch keine dieser Verbindungen expressis verbis genannt, die unter den Umfang der erfindungsgemässen Formel I fallen.

Durch die Bereitstellung der erfindungsgemässen Verbindungen der Formel I ist es nun gelungen einen wertvollen Beitrag zur Bekämpfung der beträchtliche landwirtschaftliche Schäden an Pflanzgut verursachenden Pflanzennematoden und Bodenpilzen zu leisten. Auf diese Weise können Ernteeinbussen bei Kulturpflanzen wie z.B. Kartoffeln, Getreide, Rüben, Raps, Kohl, Tabak, Sojabohnen, Baumwolle, Mais, Reis und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung

von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formel I lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Ebenso lassen sich vorzugsweise mit den Wirkstoffen der Formel I besonders schädliche Bodenpilze der Gattung Pythium, wie z.B. Pythium ultimum, sowie der Gattung Rhizoctonia, wie z.B. Rhizoctonia solani erfolgreich bekämpfen.

Zur Bekämpfung der Pflanzennematoden und Bodenpilze und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematoden- und Bodenpilz-Species und werden somit den Erfordernissen der Praxis gerecht. Die nematizide und fungizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Eine nematizide Wirksamkeit wird durch Hemmung der Wurzelgallbildung an der behandelten Kulturpflanze im Vergleich zur unbehandelten Pflanze festgestellt und auch bestimmt.

Die Wirkung wird als "gut" bezeichnet, wenn der Befall der behandelten Pflanze geringer ist als 20 % des Befalls der unbehandelten Pflanze.

Im Rahmen der vorliegenden Erfindung sind jene 2-Mercapto-5-pyrimidyl-1,3,4-oxadiazole der Formel Ia

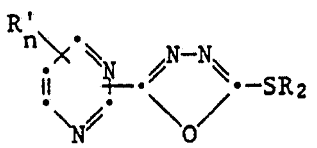

(Ia)

bevorzugt, bei welchen R′ Methyl und n = 0, 1 oder 2, $R_2$ Difluormethyl, 3,4,4-Trifluor-3-buten-1-yl, Cyanomethyl, Difluormethyl-difluormethyl oder Propargyl bedeuten.

Bevorzugt sind auch jene 2-Mercapto-5-pyrimidyl-1,3,4-thiadiazole der Formel Ib

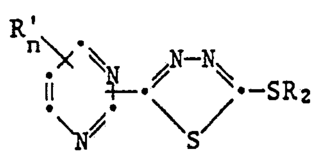

(Ib)

bei welchen $R_2$ Difluormethyl, 3,4,4-Trifluor-3-buten, Tetrafluoräthyl, Cyanomethyl, Allyl, Propargyl oder 2-Bromalkyl und n = 0 bedeuten.

Von diesen Gruppen sind folgende Verbindungen als Einzelverbindungen hervorzuheben.

- 2-Difluormethylthio-5-pyrimid-5′-yl-1,3,4-oxadiazol,
- 2-Difluormethylthio-5-pyrimid-4′-yl-1,3,4-oxadiazol,
- 2-Difluormethylthio-5-pyrimid-2′-yl-1,3,4-oxadiazol,
- 2-(3,4,4-Trifluor-3-butenthio)-5-pyrimid-5′-yl-1,3,4-oxadiazol,
- 2-(3,4,4-Trifluor-3-butenthio)-5-pyrimid-4′-yl-1,3,4-oxadiazol,
- 2-(3,4,4-Trifluor-3-butenthio)-5-pyrimid-2′-yl-1,3,4-oxadiazol,
- 2-Difluormethylthio-5-pyrimid-5′-yl-1,3,4-thiadiazol,
- 2-Difluormethylthio-5-pyrimid-4′-yl-1,3,4-thiadiazol,
- 2-Difluormethylthio-5-pyrimid-2′-yl-1,3,4-thiadiazol,
- 2-(3,4,4-Trifluor-3-butenthio)-5-pyrimid-5′-yl-1,3,4-thiadiazol,

- 2-(3,4,4-Trifluor-3-butenthio)-5-pyrimid-4′-yl-1,3,4-thiadiazol und
- 2-(3,4,4-Trifluor-3-butenthio)-5-pyrimid-2′-yl-1,3,4-thiadiazol.

Verbindungen der Formel I werden erfindungsgemäss hergestellt, indem man

a) in einer Kondensationsreaktion eine Verbindung der Formel II

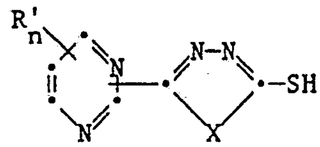

(II)

oder eine Verbindung der Formel III

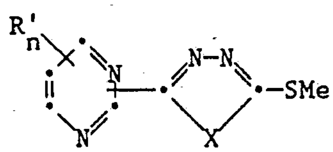

(III)

mit einer Verbindung der Formel IV

Hal-$R_2$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

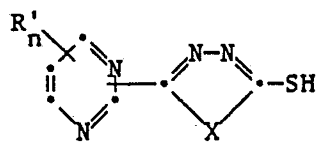

(II)

mit einer Verbindung der Formel V

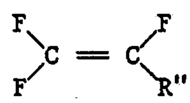

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

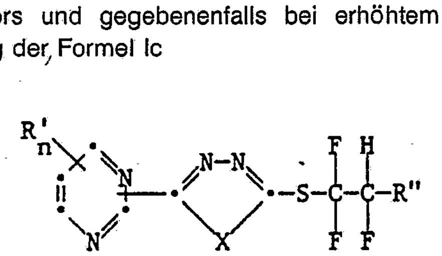

(Ic)

oder zu einer Verbindung der Formel Id

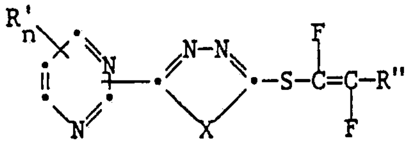

(Id)

führt, wobei in den vorstehend genannten Formeln II, III, Ic, Id, IV und V Me für ein Alkalimetall oder für Ammonium steht, Hal Halogen, vorzugsweise Chlor, Brom oder Jod bedeutet, und R″ Fluor oder Trifluormethyl darstellt, während R′ und n die unter Formel I angegebenen Bedeutungen besitzen.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkyli- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Kaliumethylat.

Die Zugabe katalytischer Mengen eines Kronenethers, wie z.B. 18-Krone-6 oder 15-Krone-5, wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylammoniumchloride oder -bromide, vorzugsweise Tetra-n-butylammoniumbromid, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10 bis 90° C, vorzugsweise 30° bis 80° C. Für die Druckverhältnisse während des Reaktionsablaufs sind 1 bis 20 bar, vorzugsweise 6 bis 14 bar, massgebend.

Die Erfindung betrifft auch Mittel, zur Bekämpfung von pflanzenschädigenden Nematoden und Pilzen sowie zur präventiven Verhütung des Nematoden- und Pilzbefalls von Pflanzgut, welche die Wirkstoffe der Formel I enthalten.

Darüber hinaus schliesst die vorliegende Erfindung zusätzlich die Herstellung nematizider und fungizider Mittel ein, die gekennzeichnet ist durch das innige Vermischen von Aktivsubstanzen der Formel I mit einer oder mehreren hierin beschriebenen Trägerstoffen und Hilfsingredienzen.

Eingeschlossen ist auch ein Verfahren zur Behandlung von Pflanzen, das durch die Applikation der Verbindungen der Formel I oder der neuen Mittel charakterisiert ist.

Ein bezvorzugtes Verfahren für den Einsatz eines Wirkstoffs der Formel I bzw. eines nematiziden und fungiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in

der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die neuen Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

## 1. Herstellungsbeispiele

### 2-Difluormethylthio-5-pyrimid-4′-yl-1,3,4-thiadiazol (Verbindung Nr. 3.8)

1 g (0,015 Mol) 85%iges Kaliumhydroxid werden in 12 ml Wasser gelöst und mit einer Lösung von 2 g (0,01 Mol) 2-Mercapto-5-pyrimid-4′-yl-1,3,4-thiadiazol in 100 ml 1,4-Dioxan versetzt. Zum Reaktionsgemisch werden 0,5 g Tetramethylammoniumchlorid als Phasentransferkatalysator gegeben. Nun werden bei 40° C während 8 Stunden ca. 1,3 g (0,015 Mol) Chlordifluormethan (Freon 22) eingeleitet. Danach wird ein Zusatz von 0,5 g 85%igem Kaliumhydroxid und 0,25 g Tetramethylammoniumchlorid gemacht und weitere 8 Stunden ca. 1,3 g Freon 22 bei 40° C eingeleitet. Nach dem Abdestillieren des Dioxans unter vermindertem Druck wird der Rückstand in ca. 100 ml Essigsäureethylester gelöst und nacheinander je 3 mal mit je ca. 50 ml Wasser, 1 N Natronlauge und Wasser extrahiert. Die organische Lösung wird mit Natriumsulfat getrocknet, filtriert und unter vermindertem Druck eingeengt. Das Rohprodukt wird über eine Flash-Kieselgelsäule mit einem Gemiscsh von 1 Teil Essigsäureethylester und 3 Teilen Hexan als Fliessmittel gereinigt. Man erhält so 0,9 g Produkt vom Smp. 113-115° C.

### 2-(3,4,4-Trifluor-3-butenthio)-5-pyrimid-4′-yl-1,3,4-oxadiazol (Verbindung Nr. 3.19)

Zu einer Lösung von 0,8 g (0,0044 Mol) 2-Mercapto-5-pyrimid-4′-yl-1,3,4-oxadiazol in einem Gemisch von 30 ml 1,4-Dioxan und 3 ml Dimethylformamid werden 1,05 g (0,0093 Mol) Kalium-tert.-butylat gegeben. Anschliessend werden 2,5 g (0,013 Mol) 4-Brom-1,1,2-trifluorbuten bei Raumtemperatur (ca. 25° C) zugetropft und 16 Stunden gerührt. Das Dioxan wird unter vermindertem Druck abdestilliert und der Rückstand in ca. 50 ml Essigsäurethylester gelöst und nacheinander je 3 mal mit je ca. 30 ml Wasser, 1N Natronlauge und Wasser extrahiert. Die organische Lösung wird mit Natriumsulfat getrocknet, filtriert und unter vermindertem Druck eingeengt. Der Rückstand wird mit Petrolether angeschlämmt, filtriert und die Kristalle bei ca. 25° C getrocknet. Man erhält so 0,6 g Produkt vom Smp. 61-63° C.

Tabelle 1

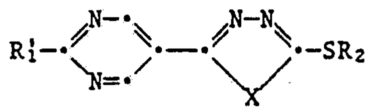

| Verb. Nr. | $R_2$ | X | $R_1'$ | Physik. Daten |
|---|---|---|---|---|
| 1.1. | $-CH_3$ | O | H | |
| 1.2 | $-CH_3$ | S | H | |
| 1.3 | $-CH_2CH=CH_2$ | O | H | |
| 1.4 | $-CH_2CH=CH_2$ | S | H | |
| 1.5 | $-CH_2C\equiv CH$ | O | H | |
| 1.6 | $-CH_2C\equiv CH$ | S | H | |
| 1.7 | $-CH_2CN$ | O | H | |
| 1.8 | $-CH_2CN$ | S | H | |
| 1.9 | $-CH_2OCH_3$ | O | H | |
| 1.10 | $-CH_2OCH_3$ | S | H | |
| 1.11 | $-CHF_2$ | O | H | |
| 1.12 | $-CHF_2$ | S | H | |
| 1.13 | $-CF_3$ | O | H | |
| 1.14 | $-CF_3$ | S | H | |
| 1.15 | $-CF_2CHF_2$ | O | H | |
| 1.16 | $-CF_2CHF_2$ | S | H | |
| 1.17 | $-CH_2C(Br)=CH_2$ | O | H | |
| 1.18 | $-CH_2C(Br)=CH_2$ | S | H | |
| 1.19 | $-CH_2CH_2CF=CF_2$ | O | H | |
| 1.20 | $-CH_2CH_2CF=CF_2$ | S | H | |
| 1.21 | H | O | H | |
| 1.22 | H | S | H | |
| 1.23 | $-CHF_2$ | O | $CH_3S$ | |
| 1.24 | $-CHF_2$ | S | $CH_3S$ | |
| 1.25 | $-CH_2CH_2CF=CF_2$ | O | $CH_3S$ | |
| 1.26 | $-CH_2CH_2CF=CF_2$ | S | $CH_3S$ | |

Tabelle 2

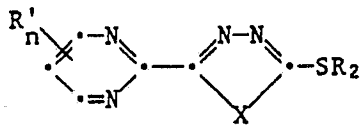

| Verb. Nr. | $R_2$ | R' | X | n | Physik. Daten |
|---|---|---|---|---|---|
| 2.1 | H | | O | 0 | Smp. 219-220°C |
| 2.2 | H | | S | 0 | Smp. >250°C |
| 2.3 | $CH_3$ | | O | 0 | |
| 2.4 | $CH_3$ | | S | 0 | |
| 2.5 | $-CH_2CH=CH_2$ | | O | 0 | |
| 2.6 | $-CH_2CH=CH_2$ | | S | 0 | |
| 2.7 | $-CH_2-C(Br)=CH_2$ | | O | 0 | |
| 2.8 | $-CH_2-C(Br)=CH_2$ | | S | 0 | |
| 2.9 | $-CH_2C\equiv CH$ | | O | 0 | |
| 2.10 | $-CH_2C\equiv CH$ | | S | 0 | |
| 2.11 | $-CH_2CN$ | | O | 0 | |
| 2.12 | $-CH_2CN$ | | S | 0 | |
| 2.13 | $-CH_2OCH_3$ | | O | 0 | |
| 2.14 | $-CH_2OCH_3$ | | S | 0 | |
| 2.15 | $-CHF_2$ | | O | 0 | Smp. 105-107°C |
| 2.16 | $-CHF_2$ | | S | 0 | Smp. 126-128°C |
| 2.17 | $-CF_2CHF_2$ | | O | 0 | |
| 2.18 | $-CF_2CHF_2$ | | S | 0 | |
| 2.19 | $-CF_3$ | | O | 0 | |
| 2.20 | $-CF_3$ | | S | 0 | |
| 2.21 | $-CH_2CH_2CF=CF_2$ | | O | 0 | $n_D^{30}$ = 1,5537 |
| 2.22 | $-CH_2CH_2CF=CF_2$ | | S | 0 | Oel |
| 2.23 | $-CHF_2$ | 4-$CH_3$ 6-$CH_3$ | O | 2 | Smp. 144-145°C |
| 2.24 | $-CHF_2$ | 4-$CH_3$ 6-$CH_3$ | S | 2 | |
| 2.25 | $-CH_2CH_2CF=CF_2$ | 4-$CH_3$ 6-$CH_3$ | O | 2 | Smp. 98-100°C |
| 2.26 | $-CH_2CH_2CF=CF_2$ | 4-$CH_3$ 6-$CH_3$ | S | 2 | Smp. 57-59°C |

Tabelle 2 (Fortsetzung)

| Verb. Nr. | $R_2$ | R' | X | n | Physik. Daten |
|---|---|---|---|---|---|
| 2.27 | $-CHF_2$ | 4-$CH_3$ 6– 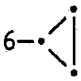 | O | 2 | |
| 2.28 | $-CHF_2$ | 4-$CH_3$ 6– 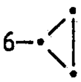 | S | 2 | |
| 2.29 | $-CH_2CH_2CF=CF_2$ | 4-$CH_3$ 6– 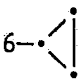 | O | 2 | |
| 2.30 | $-CH_2CH_2CF=CF_2$ | 4-$CH_3$ 6– 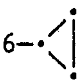 | S | 2 | |
| 2.31 | $-CHF_2$ | 4-$OCH_3$ 6-$OCH_3$ | O | 2 | |
| 2.32 | -H | 4-$CH_3$ 6-$CH_3$ | O | 2 | Smp. >240°C |
| 2.33 | -H | 4-$CH_3$ 6-$CH_3$ | S | 2 | Smp. >240°C |

Tabelle 3

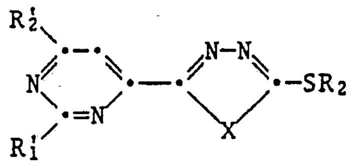

| Verb. Nr. | $R_2$ | $R_1'$ | $R_2'$ | X | Physik. Daten |
|---|---|---|---|---|---|
| 3.1 | H | H | H | O | Smp. 212-215°C |
| 3.2 | H | H | H | S | Smp. 214-216°C |
| 3.3 | $CH_3$ | H | H | O | |
| 3.4 | $CH_3$ | H | H | S | |
| 3.5 | $CF_3$ | H | H | O | |
| 3.6 | $CF_3$ | H | H | S | |
| 3.7 | $-CHF_2$ | H | H | O | Oel |
| 3.8 | $-CHF_2$ | H | H | S | Smp. 113-115°C |
| 3.9 | $-CF_2CHF_2$ | H | H | O | |
| 3.10 | $-CF_2CHF_2$ | H | H | S | |
| 3.11 | $-CH_2CN$ | H | H | O | |
| 3.12 | $-CH_2CN$ | H | H | S | |
| 3.13 | $-CH_2OCH_3$ | H | H | O | |

Tabelle 3 (Fortsetzung)

| Verb. Nr. | $R_2$ | $R_1'$ | $R_2'$ | X | Physik. Daten |
|---|---|---|---|---|---|
| 3.14 | $-CH_2OCH_3$ | H | H | S | |
| 3.15 | $-CH_2CH=CH_2$ | H | H | O | |
| 3.16 | $-CH_2CH=CH_2$ | H | H | S | |
| 3.17 | $-CH_2-C(Br)=CH_2$ | H | H | O | |
| 3.18 | $-CH_2-C(Br)=CH_2$ | H | H | S | |
| 3.19 | $-CH_2CH_2CF=CF_2$ | H | H | O | Smp. 61-63°C |
| 3.20 | $-CH_2CH_2CF=CF_2$ | H | H | S | Smp. 88-90°C |
| 3.21 | $-CH_2C\equiv CH$ | H | H | O | |
| 3.22 | $-CH_2C\equiv CH$ | H | H | S | |
| 3.23 | $-CHF_2$ | $CH_3$ | $CH_3$ | O | |
| 3.24 | $-CHF_2$ | $CH_3$ | $CH_3$ | S | |
| 3.25 | $-CHF_2$ | $CH_3O$ | $CH_3O$ | O | |
| 3.26 | $-CHF_2$ | $CH_3O$ | $CH_3O$ | S | |
| 3.27 | $-CH_2CH_2CF=CF_2$ | $CH_3O$ | $CH_3O$ | O | |
| 3.28 | $-CH_2CH_2CF=CF_2$ | $CH_3O$ | $CH_3O$ | S | |
| 3.29 | $-CH_2CH_2CF=CF_2$ | $CH_3$ | $CH_3$ | O | |
| 3.30 | $-CH_2CH_2CF=CF_2$ | $CH_3$ | $CH_3$ | S | |
| 3.31 | $-CHF_2$ | $CH_3O$ | $CH_3$ | O | |
| 3.32 | $-CHF_2$ | $CH_3S$ | $CH_3$ | O | |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen 1-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen 1-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1-3 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1-3 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 2.8 Extruder Granulat | |
|---|---|
| Wirkstoff aus den Tabellen 1-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen 1-3 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen 1-3 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### 3.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26±1 °C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Die Verbindungen aus den Tabellen 1-3 zeigen gegen Meloidogyne incognita durch Reduktion der Wurzelgallbildung eine Wirksamkeit. Unbehandelte jedoch infizierte Kontrollpflanzen weisen dagegen starke Wurzelgallbildung auf (= 100 %). Eine gute Wirkung zeigen z.B. die Verbindungen Nr. 1.11, 1.12, 1.19, 1.20, 2.15, 2.16, 2.21 und 2.22 (weniger als 20 % Restbefall), die Verbindungen Nr. 3.7, 3.8, 3.19 und 3.20 hemmen im obigen Versuch die Wurzelgallbildung sogar fast vollständig (0-10 % Restbefall).

### 3.2 Pythium ultimum auf Beta vulgaris (Zuckerrübe, cv. Kleinwanzleben Monogerm) und auf Pythium ultimum auf Zea mays (Mais, cv. Sweet Corn)

Testprinzip: Bodenpilz: protektiv lokale Bodenapplikation.

Testmethode: Myzel von Pythium ultimum wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10 °C werden pro Schale 10 Körner der zu prüfenden Pflanze (Mais oder Zuckerrübe) gesteckt. Am nächsten Tag werden die so vorbereiteten Schalen mit je 50 ml, aus 25 % Spritzpulver und Wasser hergestellten Spritzlösungen mit 20; 6; 2; 0,6; 0,2; 0,06 und 0,02 ppm AS übergossen. Nach einer 7-tägigen Inkubationsphase bei 10 °C und einer anschliessenden 4-tägigen In kubationsphase bei 22 °C wird die Wirkung der Testsubstanzen durch zahlenmässige Bestimmung des Auflaufs der Testpflanzen nach dem folgenden Notenschema bewertet:

| mikrobizide Aktivität % | Bewertungsnote |
|---|---|
| > 95 | 1 |
| 80-95 | 3 |
| 50-80 | 6 |
| 0-50 | 9 (=wirkungslos) |

Verbindungen aus den Tabellen 1-3 zeigen gegen Pythium ultimum gute Wirkung. So zeigen die Verbindungen 3.1 und 3.19 eine Wirkung von mehr als 80 %.

3.3 Wirkung gegen Rhizoctonia solani (Bodenpilz auf Reispflanzen) Protektiv-lokale Bodenapplikation

12 Tage alte Reispflanzen werden mit einer aus einer Formulierung des Wirkstoffes hergestellten Spritzbrühe (0,002 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27 °C (Tag), bzw. 23 °C (Nacht) und 100 % rel. Luftfeuchtigkeit (Feuchtkasten) in der Klimakammer wird der Pilzbefall auf Blattscheide, Blättern und Stengel beurteilt.

Verbindungen aus den Tabellen 1-3 zeigen gegen Rhizoctonia solani gute Aktivität. So zeigt die Verbindung 3.19 eine Wirkung von ca. 80 %.

**Ansprüche**

1. 2-Mercapto-5-pyrimidyl-1,3,4-oxadiazole und 2-Mercapto-5-pyrimidyl-1,3,4-thiadiazole der allgemeinen Formel

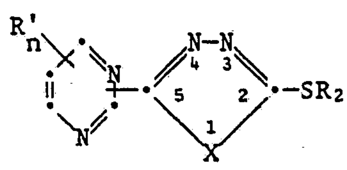

(I)

in welcher
X Sauerstoff oder Schwefel
R′ Halogen, $CF_3$, $C_1$-$C_3$-Alkyl, $C_1$-$C_3$-Alkoxy, $C_1$-$C_3$-Alkylthio, Cyclopropyl und n eine Zahl von 0 bis 3,
$R_2$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substitui3241s $C_3$-$C_7$-Alkinyl, bedeuten sowie Salze dieser Verbindungen.

2. 2-Mercapto-5-pyrimidyl-1,3,4-oxadiazole der allgemeinen Formel Ia

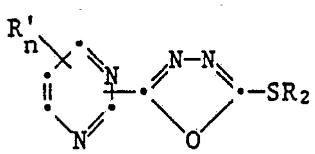

(Ia)

gemäss Anspruch 1, bei welchen R′ Methyl und n = 0, 1 oder 2, $R_2$ Difluormethyl, 3,4,4-Trifluor-3-buten-1-yl, Cyanomethyl, Difluormethyl-difluormethyl oder Propargyl bedeuten.

3. 2-Mercapto-5-pyrimidyl-1,3,4-thiadiazole der allgemeinen Formel Ib

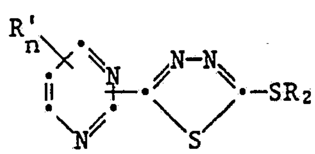

(Ib)

gemäss Anspruch 1, bei welchen $R_2$ Difluormethyl, 3,4,4-Trifluor-3-buten-1-yl, Tetrafluoräthyl, Cyanomethyl, Allyl, Propargyl oder 2-Bromalkyl und n = 0 bedeuten.

4. Eine Verbindung gemäss Anspruch 2 ausgewählt aus der Gruppe bestehend aus
- 2-Difluormethylthio-5-pyrimid-5′-yl-1,3,4-oxadiazol,

- 2-Difluormethylthio-5-pyrimid-4′-yl-1,3,4-oxadiazol,
- 2-Difluormethylthio-5-pyrimid-2′-yl-1,3,4-oxadiazol,
- 2-(3,4,4-Trifluor-3-buten-1-ylthio)-5-pyrimid-5′-yl-1,3,4-oxadiazol,
- 2-(3,4,4-Trifluor-3-buten-1-ylthio)-5-pyrimid-4′-yl-1,3,4-oxadiazol,
- 2-(3,4,4-Trifluor-3-buten-1-ylthio)-5-pyrimid-2′-yl-1,3,4-oxadiazol.

5. Eine Verbindung gemäss Anspruch 3, ausgewählt aus der Gruppe bestehend aus

- 2-Difluormethylthio-5-pyrimid-5′-yl-1,3,4-thiadiazol,
- 2-Difluormethylthio-5-pyrimid-4′-yl-1,3,4-thiadiazol,
- 2-Difluormethylthio-5-pyrimid-2′-yl-1,3,4-thiadiazol,
- 2-(3,4,4-Trifluor-3-buten-1-ylthio)-5-pyrimid-5′-yl-1,3,4-thiadiazol,
- 2-(3,4,4-Trifluor-3-buten-1-ylthio)-5-pyrimid-4′-yl-1,3,4-thiadiazol und
- 2-(3,4,4-Trifluor-3-buten-1-ylthio)-5-pyrimid-2′-yl-1,3,4-thiadiazol.

6. Verfahren zur Herstellung der Verbindungen der Formel I gemäss Anspruch 1, dadurch gekennzeichnet, dass man a) in einer Kondensationsreaktion eine Verbindung der Formel II

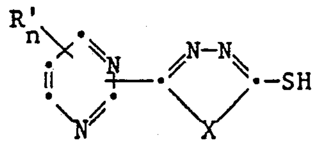

(II)

oder eine Verbindung der Formel III

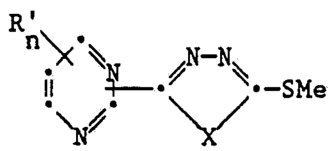

(III)

mit einer Verbindung der Formel IV

Hal-$R_2$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

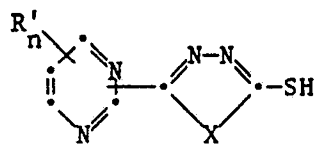

(II)

mit einer Verbindung der Formel V

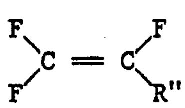

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

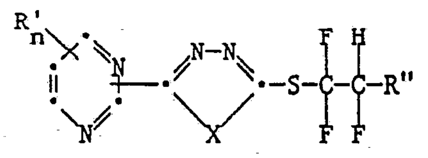

(Ic)

oder zu einer Verbindung der Formel Id

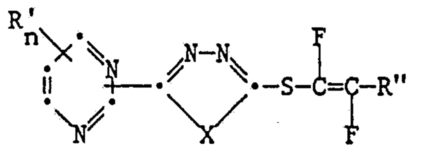

(Id)

führt, wobei in den vorstehend genannten Formeln II, III, Ic, Id, IV und V Me für ein Alkalimetall oder für Ammonium steht, Hal Halogen, vorzugsweise Chlor, Brom oder Jod bedeutet, und R″ Fluor oder Trifluormethyl darstellt, während R′ und n die unter Formel I angegebenen Bedeutungen besitzen.

7. Schädlingsbekämpfungsmittel zur Bekämpfung oder Verhütung des Befalls von Pflanzen durch Nematoden und Pilze, dadurch gekennzeichnet, dass es als aktive Komponente mindestens eine Verbindung der Formel I, gemäss Anspruch 1 enthält.

8. Mittel gemäss Anspruch 7, dadurch gekennzeichnet, dass es als aktive Komponente mindestens eine Verbindung der Formel I gemäss der Ansprüche 2 bis 5 enthält.

9. Mittel gemäss Anspruch 7, dadurch gekennzeichnet, dass es als aktive Komponente 2-Difluormethylthio-5-pyrimid-4′yl-1,3,4-thiadiazol enthält.

10. Mittel gemäss Anspruch 7, dadurch gekennzeichnet, dass es als aktive Komponente 2-(3,4,4-Trifluor-3-buten-1-ylthio)-5-pyrimid-4′yl-1,3,4-oxadiazol enthält.

11. Mittel nach einem der Ansprüche 7 bis 10, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

12. Mittel nach Anspruch 11, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

13. Verfahren zur Herstellung eines agrochemischen Mittels, dadurch gekennzeichnet, dass mindestens eine Verbindung der Formel I gemäss Anspruch 1 mit geeigneten festen oder flüssigen Zusatzstoffen und Tensiden innig vermischt.

14. Verfahren zur Bekämpfung oder Verhütung eines Befalls von Kulturpflanzen durch Nematoden und Pilze, dadurch gekennzeichnet, dass man eine Verbindung der Formel I gemäss Anspruch 1 auf die Pflanze oder deren Standort appliziert.

15. Verfahren gemäss Anspruch 14, von Verbindungen der Formel I gemäss einem der Ansprüche 2 bis 5.

16. Verfahren gemäss Anspruch 15, dadurch gekennzeichnet, dass es sich bei den Nematoden und Pilzen um pflanzenparasitäre Arten handelt.

17. Verfahren gemäss Anspruch 16 gegen Nematoden der Gattung Meloidogyne, Heterodera oder Globodera.

18. Verwendung gemäss Anspruch 16 gegen Pilze der Gattung Pythium oder Rhizoctonia.

Patentansprüche für folgenden Vertragsstaat: ES

1. Verfahren zur Herstellung der 2-Mercapto-5-pyrimidyl-1,3,4-oxadiazole und 2-Mercapto-5-pyrimidyl-1,3,4-thiadiazole der allgemeinen Formel

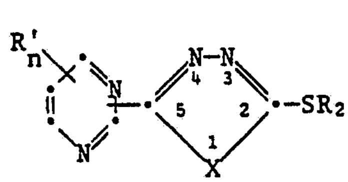

(I)

in welcher
X Sauerstoff oder Schwefel
R′ Halogen, $CF_3$, $C_1$-$C_3$-Alkyl, $C_1$-$C_3$-Alkoxy, $C_1$-$C_3$-Alkylthio, Cyclopropyl und n eine Zahl von 0 bis 3,
$R_2$ Wasserstoff, unsubstituiertes oder durch Halogen, $C_1$-$C_3$-Alkoxy oder Cyano substituiertes $C_1$-$C_5$-Alkyl; unsubstituiertes oder durch Halogen substituiertes $C_3$-$C_7$-Alkenyl; unsubstituiertes oder durch Halogen substituiertes $C_3$-$C_7$-Alkinyl, dadurch gekennzeichnet, dass man

a) in einer Kondensationsreaktion eine Verbindung der Formel II

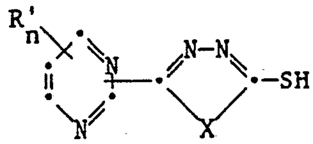

(II)

oder eine Verbindung der Formel III

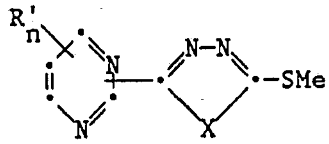

(III)

mit einer Verbindung der Formel IV

$Hal-R_2$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

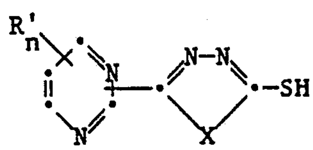

(II)

mit einer Verbindung der Formel V

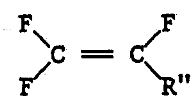

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

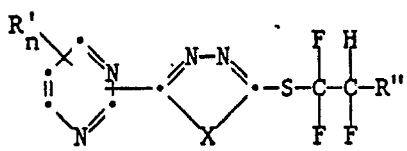

(Ic)

oder zu einer Verbindung der Formel Id

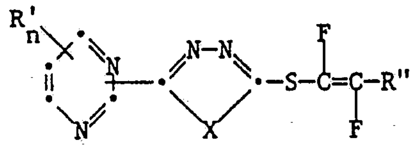

(Id)

führt, wobei in den vorstehend genannten Formeln II, III, Ic, Id, IV und V Me für ein Alkalimetall oder für Ammonium steht, Hal Halogen, vorzugsweise Chlor, Brom oder Jod bedeutet, und R″ Fluor oder Trifluormethyl darstellt, während R′ und n die unter Formel I angegebenen Bedeutungen besitzen.

2. Verfahren zur Herstellung der 2-Mercapto-5-pyrimidyl-1,3,4-oxadiazole der allgemeinen Formel Ia

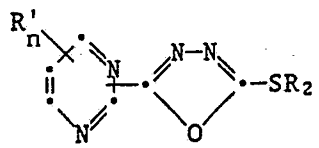

(Ia)

gemäss Anspruch 1, dadurch gekennzeichnet, dass R′ Methyl und n = 0, 1 oder 2, $R_2$ Difluormethyl, 3,4,4-Trifluor-3-buten-1-yl, Cyanomethyl, Difluormethyl-difluormethyl oder Propargyl bedeuten.

3. Verfahren zur Herstellung der 2-Mercapto-5-pyrimidyl-1,3,4-thiadiazole der allgemeinen Formel Ib

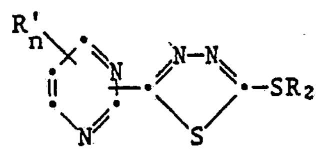

(Ib)

gemäss Anspruch 1, dadurch gekennzeichnet, dass $R_2$ Difluormethyl, 3,4,4-Trifluor-3-buten-1-yl, Tetrafluoräthyl, Cyanomethyl, Allyl, Propargyl oder 2-Bromalkyl und n = 0 bedeuten.

4. Verfahren gemäss Anspruch 2 zur Herstellung einer Verbindung ausgewählt aus der Gruppe bestehend aus

- 2-Difluormethylthio-5-pyrimid-5′-yl-1,3,4-oxadiazol,
- 2-Difluormethylthio-5-pyrimid-4′-yl-1,3,4-oxadiazol,
- 2-Difluormethylthio-5-pyrimid-2′-yl-1,3,4-oxadiazol,
- 2-(3,4,4-Trifluor-3-buten-1-ylthio)-5-pyrimid-5′-yl-1,3,4-oxadiazol,
- 2-(3,4,4-Trifluor-3-buten-1-ylthio)-5-pyrimid-4′-yl-1,3,4-oxadiazol,
- 2-(3,4,4-Trifluor-3-buten-1-ylthio)-5-pyrimid-2′-yl-1,3,4-oxadiazol.

5. Verfahren gemäss Anspruch 3 zur Herstellung einer Verbindung ausgewählt aus der Gruppe bestehend aus

- 2-Difluormethylthio-5-pyrimid-5′-yl-1,3,4-thiadiazol,
- 2-Difluormethylthio-5-pyrimid-4′-yl-1,3,4-thiadiazol,
- 2-Difluormethylthio-5-pyrimid-2′-yl-1,3,4-thiadiazol,
- 2-(3,4,4-Trifluor-3-buten-1-ylthio)-5-pyrimid-5′-yl-1,3,4-thiadiazol,
- 2-(3,4,4-Trifluor-3-buten-1-ylthio)-5-pyrimid-4′-yl-1,3,4-thiadiazol und
- 2-(3,4,4-Trifluor-3-buten-1-ylthio)-5-pyrimid-2′-yl-1,3,4-thiadiazol.